# EUROPÄISCHE PATENTSCHRIFT

(11) **EP 0 829 466 B1**
(45) Veröffentlichungstag und Bekanntmachung des Hinweises auf die Patenterteilung: **12.12.2001**
(21) Anmeldenummer: 97115718.5
(22) Anmeldetag: 10.09.1997
(51) Int. Cl.: C07C 67/04, C07C 69/06, C07C 67/03, C07C 35/06, C07C 27/02

(54) **Verfahren zur Herstellung von Cyclopentanolen**
Process for the preparation of cyclopentanols
Procédé de préparation de cyclopentanols

(30) Priorität: 13.09.1996 DE 19637429
(43) Veröffentlichungstag der Anmeldung: 18.03.1998
(73) Patentinhaber: BASF AKTIENGESELLSCHAFT, 67056 Ludwigshafen (DE)
(72) Erfinder: Fischer, Rolf, Dr., 69121 Heidelberg (DE); Pinkos, Rolf, Dr., 67098 Bad Dürkheim (DE); Rieber, Norbert, Dr., 68259 Mannheim (DE); Schulz, Michael, Dr., 67067 Ludwigshafen (DE); Teles, Joaquim Henrique, Dr., 67059 Ludwigshafen (DE)
(74) Vertreter: Isenbruck, Günter, Dr.

(56) Entgegenhaltungen:
- EP-A- 0 476 400
- EP-A- 0 548 763

## Beschreibung

Die Erfindung betrifft ein Verfahren zur Herstellung von gegebenenfalls substituierten Cyclopentanolen. Weiterhin betrifft die Erfindung ein Verfahren zur Herstellung von gegebenenfalls substituierten Cyclopentylformiaten, aus denen die Cyclopentanole hergestellt werden.

Zur Herstellung von Cyclopentylverbindungen wird in der Regel Cyclopentanon benutzt, das durch Ruzika-Cyclisierung von Adipinsäure oder Adipinsäureestern gewonnen werden kann. Das so erhaltene Cyclopentanon wird dann in weiteren Umsetzungen eingesetzt. Die Synthese hat den Nachteil, daß mehr als 40% des Einsatzstoffes in Form von Wasser und Kohlendioxid verloren gehen.

Es besteht somit Nachfrage nach anderen Verfahren zur Herstellung von Cyclopentylverbindungen, unter besserer Nutzung der Ausgangsstoffe.

Verfahren zur Herstellung bestimmter cyclischer und acyclischer Alkanole sind bekannt. Aus US 3,527,816 ist ein Verfahren zur Herstellung von olefinischen Alkoholen bekannt. Dabei werden Triolefine, in denen zwei gegebenenfalls substituierte Cyclohexenringe über eine Etheneinheit verbunden sind, mit aliphatischen Carbonsäuren wie Ameisensäure umgesetzt und die entstehenden Ameisensäureester zu den entsprechenden Alkoholen gespalten.

Aus H.L. Wunderly, F.J. Sowa, J. Am. Chem. Soc., 59 (1973), Seiten 1010 bis 1011 ist die Addition von Essigsäure an Cyclohexen in Gegenwart einer Essigsäure-Borfluorid-Verbindung beschrieben, wobei die Menge an eingesetztem Borfluorid eine deutliche Auswirkung auf die Menge des erhaltenen Esters hat. Es wird dabei mit hohen Borfluorid-Konzentrationen und langen Reaktionszeiten von 50 bis 220 Stunden gearbeitet. Bei der Umsetzung tritt Polymerisation von Cyclohexen als Nebenreaktion auf.

Aus T.B. Dorris, F.J. Sowa, J.A. Nieuwland, J. Am. Chem. Soc., 56 (1934), Seiten 2689 bis 2690 ist die Kondensation von Propylen mit Essigsäure, Mono-, Di-, und Trichloressigsäure sowie Benzoesäuren bekannt, die zu den entsprechenden Isopropylestern führt.

Aus P.E. Peterson, E.V.P. Tao, J. Org. Chem., 29 (1964), Seiten 2322 bis 2325 ist die Addition von Essigsäure, Ameisensäure und Trifluoressigsäure an verzweigte Alkene bekannt. Dabei handelt es sich um Alkene mit drei Alkylsubstituenten an der Doppelbindung, die in einer Gleichgewichtsreaktion mit den Säuren reagieren. Entsprechend gering sind die Ausbeuten an Estern und Alkoholen. Die Ausbeute für 1-Methylcyclopentanol beträgt etwa 50%.

Aus der GB-B 1,153,468 ist die Herstellung von Cyclooctylverbindungen bekannt, insbesondere Cyclooctylformiat und Cyclooctanol, sowie Cyclooctanon. Dabei wird Cycloocten mit Ameisensäure zum Cyclooctylformiat umgesetzt, das nachfolgend zum Alkohol gespalten wird.

EP-A 0 476 400 betrifft ein Verfahren zur Abtrennung von Cyclohexen aus Gemischen mit Benzol und Cyclohexan. Dabei werden die genannten Gemische in Gegenwart von festen stark-sauren Katalysatoren mit Carbonsäuren und Wasser bei erhöhter Temperatur in flüssiger Phase umgesetzt. Hierbei wird Cyclohexen zu Cyclohexanol und Cyclohexylcarbonsäureestern umgesetzt, die aus dem Gemisch abgetrennt werden können. Als Säurekomponente kann beispielsweise Ameisensäure eingesetzt werden. Als feste stark-saure Katalysatoren werden Heteropolysäuren, stark-saure Ionentauscher, Silikate und natürliche oder synthetisch hergestellte Zeolithe aufgeführt.

In Raffinerie- oder Cracker-Schnitten, insbesondere in C₅-Schnitten ist Cyclopenten als eine Nebenkomponente enthalten. Die Komponente kann destillativ angereichert werden. Das so erhaltene Cyclopenten wurde bislang als sogenannter C₅-Schnitt als Zusatz für Ottokraftstoffe verwendet. Eine Aufgabe der vorliegenden Erfindung ist die Bereitstellung eines Verfahrens zur Herstellung von Cyclopentylformiaten sowie Cyclopentanolen.

Eine weitere Aufgabe der Erfindung ist die Bereitstellung eines wirtschaftlichen Verfahrens zur Herstellung von Cyclopentylformiaten und Cyclopentanolen, bei dem keine Koppelprodukte entstehen und die Ausgangsstoffe effizient genutzt werden. Die Umsetzungszeit soll dabei kurz sein und der Einsatz großer Mengen an Bortrifluorid sowie die Entstehung von Nebenprodukten sollen vermieden werden.

Diese Aufgaben werden gelöst durch ein Verfahren zur Herstellung von Cyclopentylformiaten der allgemeinen Formel (II) 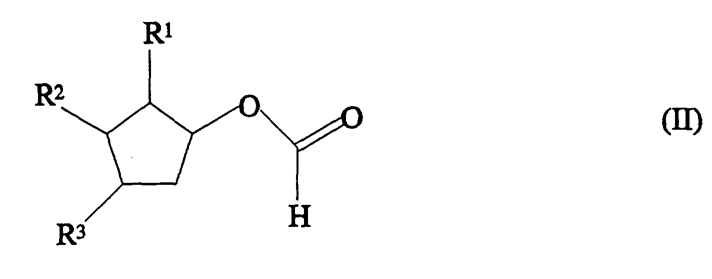 durch Umsetzung von Cyclopentenen der allgemeinen Formel (I) 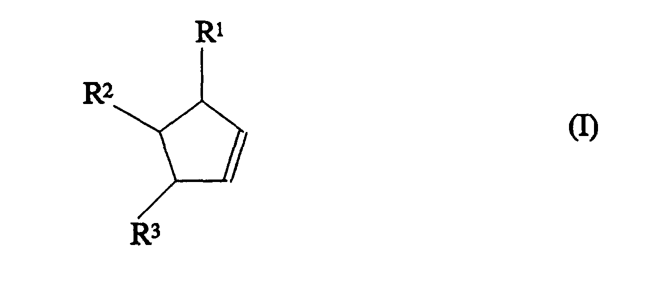 wobei R¹, R² und R³ unabhängig voneinander Wasserstoffatome oder C₁₋₈-, vorzugsweise C₁₋₄-Alkylreste sind, mit Ameisensäure in Gegenwart eines Säure-Katalysators, der ausgewählt ist aus Lewis-Säuren der allgemeinen Formel BX₃, AlX₃, SnX₄, FeX₃, MgX₂ oder ZnX₂, wobei X ein Halogenid oder der Rest C₆F₅ ist. Für die Halogenide kommen erfindungsgemäß insbesondere Fluor, Chlor, Brom und Iod in Betracht, wie auch Mischhalogenide daraus.

Die Metallhalogenide sind dabei gemäß einer Ausführungsform der Erfindung wasserfrei.

Besonders bevorzugt werden BF₃ und ZnCl₂ eingesetzt. Die Lewis-Säuren können in Form von Addukten eingesetzt werden, beispielsweise mit Ethern oder Alkoholen. Vorzugsweise werden die Alkylether, wie Diethylether oder Alkanole, wie Methanol eingesetzt. Besonders bevorzugt wird das BF₃-Diethylether-Addukt BF₃· OEt₂ eingesetzt.

Ferner können als Katalysatoren Metallkomplexe der allgemeinen Formel (R₃P)MX eingesetzt werden, wobei M Kupfer, Silber oder Gold bedeuten. X ist ein Halogenid, beispielsweise Fluorid, Chlorid, Bromid oder Iodid, Nitrat, Sulfat, Carboxylat oder Tosylat. R ist ein C₁₋₁₂-, vorzugsweise C₁₋₄-, insbesondere C₁-Alkylrest, C₆₋₁₂-, vorzugsweise C₆₋₈-Arylrest, C₁₋₁₂-O-, vorzugsweise C₁₋₄-O-, insbesondere C₁-O-Alkylrest oder C₆₋₁₂-O-, vorzugsweise C₆₋₈-O-Arylrest. Vorzugsweise ist der Rest R ein Methylrest und X Iodid. Beispiele erfindungsgemäßer bevorzugter Verbindungen sind dabei (MeO)₃PCuI und Me₃PAgI.

Weiterhin werden diese Aufgaben gelöst durch ein Verfahren zur Herstellung von Cyclopentanolen der allgemeinen Formel (III) 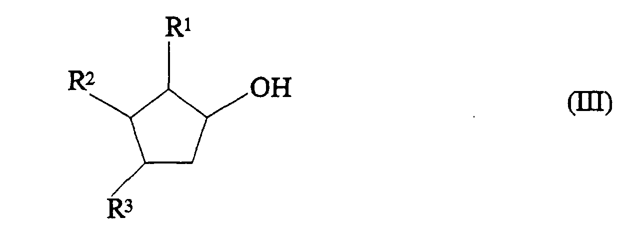 durch Umsetzung der erhaltenen Cyclopentylformiate mit einer Verbindung der allgemeinen Formel R⁴ₙXH wobei X ein Stickstoffatom ist und n den Wert 2 hat oder X ein Sauerstoffatom ist und n den Wert 1 hat und R⁴, gegebenenfalls unabhängig voneinander ein Wasserstoffatom, ein linearer oder verzweigter C₁₋₁₂-Alkylrest oder ein C₇₋₁₂-Aralkylrest ist.

Erfindungsgemäß wurde gefunden, daß Cyclopentene wie vorstehend beschrieben zu Cyclopentylformiaten und Cyclopentanolen umgesezt werden können. Dabei wird praktisch keine Bildung von Polymeren beobachtet. Darüber hinaus führt die Umsetzung in kurzer Zeit zu hohen Ausbeuten, wobei auf den Einsatz großer Mengen von Bortrifluorid verzichtet werden kann. Im Unterschied zum Einsatz der analog Wunderly und Sowa eingesetzen Essigsäure und Cyclohexen führt die erfindungsgemäße Umsetzung von Cyclopenten und Ameisensäure zu hohen Umsätzen in kurzer Zeit. Insbesondere wurde gefunden, daß C₅-Ströme aus einem (Steam)cracker oder einer Raffinerie eingesetzt werden können. Im Unterschied dazu führt die Verwendung eines C₆-Stroms aus Cyclohexen und Cyclohexan bei der Umsetzung mit Essigsäure zu sehr geringen Umsätzen.

Bei der Raffination oder beim Cracken, insbesondere Steamcracken von Erdöl oder Erdölfraktionen werden unterschiedliche Schnitte bzw. Ströme erhalten, die vornehmlich Kohlenwasserstoffe mit einer bestimmten Anzahl an Kohlenstoffatomen enthalten. So sind aus Raffinerie- oder Crackerströmen sogenannte C₅-Schnitte erhältlich. Diese C₅-Schnitte enthalten einen Anteil an Cyclopenten neben vornehmlich n-Pentan, Pentanisomeren und Pentenisomeren sowie Cyclopentan. Ein typischer C₅-Strom, der bei der Aufarbeitung von Steamcrackergemischen in einem Seitenabzug der C₅-Destillationskolonne erhalten werden kann, hat die nachstehende Zusammensetzung:

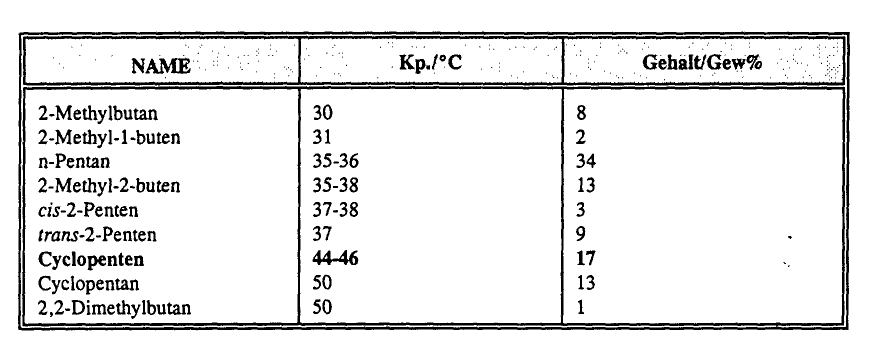

Das Gemisch enthält als Hauptkomponenten n-Pentan, 2-Methyl-2-buten, Cyclopentan und Cyclopenten. Dieses Gemisch läßt sich destillativ weiter auftrennen. Dabei werden die leichtsiedenden Komponenten abdestilliert, vorzugsweise so weit, daß der Gehalt an 2-Pentenen im als Sumpf verbleibenden Gemisch maximal 10, vorzugsweise maximal 3, insbesondere maximal 1 Gew.-% beträgt. Das Sumpfgemisch hat dabei beispielsweise die nachstehende Zusammensetzung:

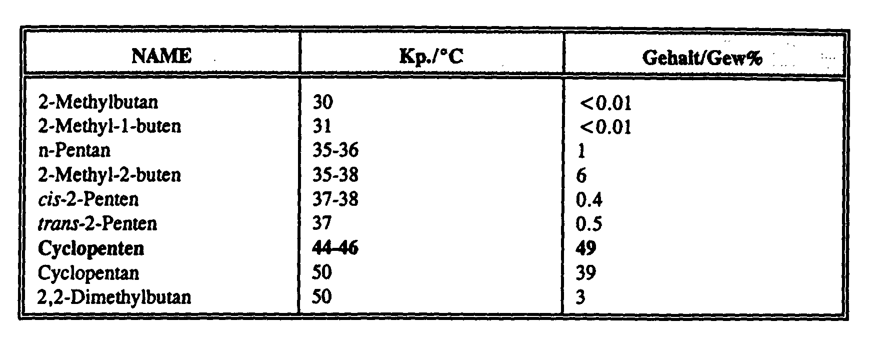

Das Sumpfprodukt besteht somit hauptsächlich aus Cyclopenten und Cyclopentan.

Im erfindungsgemäßen Verfahren kann reines Cyclopenten eingesetzt werden, das beispielsweise aus dem obigen Sumpfprodukt gewonnen werden kann. Gemäß einer Ausführungsform der Erfindung wird das Cyclopenten in Form eines C₅-Stroms aus einem (Steam)cracker oder einer Raffinerie eingesetzt. Dabei beträgt der Gehalt an Cyclopenten(en) vorzugsweise 20 bis 100, insbesondere 40 bis 80 Gew.-%. Der Gehalt an 2-Pentenen sollte dabei vorzugsweise maximal 3, insbesondere maximal 1 Gew.-% betragen.

Gemäß einer bevorzugten Ausführungsform der Erfindung wird ein Gemisch eines C₅-Stroms aus einem (Steam)cracker oder einer Raffinerie eingesetzt, bei dem leichter flüchtige Komponenten, insbesondere mit einem Siedepunkt im Bereich von 30 bis 40°C, durch Destillation weitgehend entfernt wurden. Ein solches Gemisch hat vorzugsweise einen Gehalt an Cyclopenten(en) von 20 bis 100, insbesondere 40 bis 80 Gew.-%. Der Gehalt an 2-Pentenen beträgt vorzugsweise maximal 3, insbesondere maximal 1 Gew.-%.

Nicht-cyclische Olefine, die an der Doppelbindung mono-, di- oder trisubstituiert sind, wie sie z.B. in den Cracker- oder Raffinerieschnitten vorkommen, können ebenfalls mit Ameisensäure reagieren unter Bildung von Alkylformiaten. Eine Abtrennung dieser unerwünschten Nebenprodukte kann dabei gegebenenfalls nötig sein, wenn Cyclopentylformiat in möglichst reiner Form erhalten werden soll.

Das Cyclopenten kann bzw. die Cyclopentene können somit erfindungsgemäß in reiner Form oder in Form von Gemischen eingesetzt werden, wobei in einem gegebenen Ausgangsgemisch der Gehalt an Cyclopentenen gegebenenfalls durch Abdestillieren bzw. Abtrennen von leichter siedenden Komponenten erhöht werden kann.

In einem ersten Schritt werden die Cyclopentene bzw. cyclopentenhaltigen Kohlenwasserstoffgemische, wie C₅-Ströme mit Ameisensäure zu den entsprechenden Cyclopentylformiaten umgesetzt. Die eingesetzten Cyclopentene weisen dabei die in der nachstehenden allgemeinen Formel (I) wiedergegebene Struktur auf: 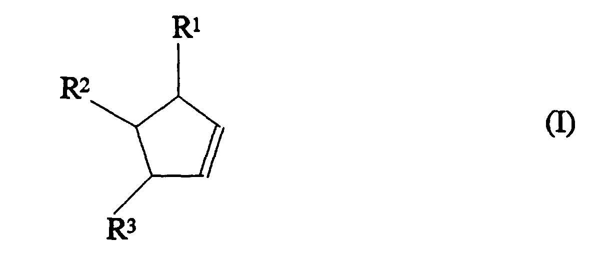 R¹, R² und R³ sind dabei unabhängig voneinander Wasserstoffatome oder C₁₋₈, vorzugsweise C₁₋₄-Alkylreste. Die Alkyreste können dabei geradkettig oder verzweigt sein. Besonders bevorzugt sind alle Reste Wasserstoffatome oder 1 bis 3 der Reste Methyl-, Ethyl-, n-Propyl-, i-Propyl-, n-Butyl-, i-Butyl- oder tert.-Butylreste, wobei die übrigen Reste Wasserstoffatome sind. Besonders bevorzugt sind alle Reste Wasserstoffatome oder 1 bis 3 Reste R¹, R² und R³ Methylreste, wobei die übrigen Reste Wasserstoffatome sind. Insbesondere sind alle Reste R¹, R² und R³ Wasserstoffatome, d.h. es wird Cyclopenten eingesetzt.

In der Beschreibung bezieht sich der Ausdruck "Cyclopentene" auf die Cyclopentene der allgemeinen Formel (I). Entsprechend beziehen sich die Ausdrücke "Cyclopentenformiate" und "Cyclopentanole" auf die entsprechenden Verbindungen der allgemeinen Formel (II) und (III).

Die Cyclopentylformiate weisen die allgemeine Formel (II) auf 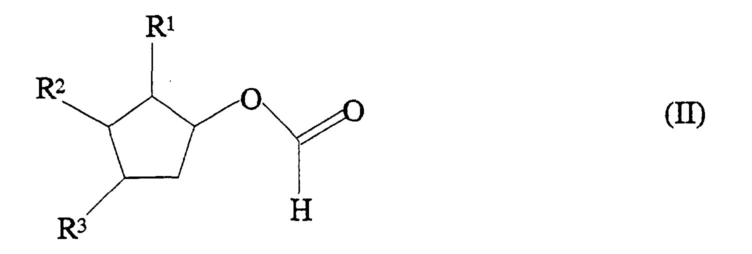 in der die Reste die vorstehend angegebene Bedeutung haben. Die Umsetzung erfolgt in Gegenwart eines homogenen oder heterogenen Säure-Katalysators.

Der Katalysator wird erfindungsgemäß in einer Menge von 0,0001 bis 25 mol%, vorzugsweise 0,005 bis 5 mol%, insbesondere 0,01 bis 1 mol%, bezogen auf die Gesamtmenge an Cyclopenten eingesetzt.

Insbesondere bevorzugt wird BF₃·OEt₂ in einer Menge von 0,02 bis 0,5 mol% eingesetzt.

Das Verhältnis der Mengen von Ameisensäure zu Cyclopenten kann ebenfalls in einem weiten Bereich variiert werden. Gemäß einer Ausführungsform der Erfindung wird mit einem Überschuß an Ameisensäure gearbeitet, um einen möglichst vollständigen Cyclopentenumsatz zu erreichen. Das Molverhältnis von Ameisensäure zu Cyclopenten liegt gemäß einer Ausführungsform der Erfindung im Bereich von 0,1 bis 25, vorzugsweise 1 bis 10, insbesondere 1,5 bis 4.

Die Umsetzung kann unter üblichen Temperaturen und Drücken durchgeführt werden.

Gemäß einer Ausführungsform der Erfindung wird die Umsetzung bei einer Temperatur von -80 bis +200°C, vorzugsweise 0°C bis 150°C, insbesondere 35°C bis 80°C durchgeführt. Gemäß einer Ausführungsform wird die Umsetzung bei einer Temperatur von etwa 80°C unter Eigendruck der Reaktanden durchgeführt.

Das bei der Umsetzung erhaltene Roh-Cyclopentylformiat kann durch Destillation gereinigt werden. Dabei werden Ameisensäure und gegebenenfalls vorliegende weitere Kohlenwasserstoffe oder Formiate entfernt. Wird beispielsweise ein Gemisch eines C₅-Stroms aus einem (Steam)cracker oder einer Raffinerie eingesetzt, so werden nach der Umsetzung die verbleibenden Kohlenwasserstoffe gemäß einer Ausführungsform der Erfindung abdestilliert.

Gemäß einer weiteren Ausführungsform der Erfindung wird bei Verwendung eines Gemisches eines C₅-Stroms das Reaktionsgemisch extrahiert mit dem im Gemisch vorhandenen Cyclopentan. Cyclopentan eignet sich gut für diese Extraktion, da die Löslichkeit von Ameisensäure in Cyclopentan nur 0,2 Gew.-% beträgt. Die Löslichkeit von Ameisensäure in n-Pentan beträgt beispielsweise bei Raumtemperatur 1,7 Gew.-%. Somit kann direkt ein ameisensäurearmes Cyclopentylformiat in der Cyclopentanphase gewonnen werden. Das so gewonnene Roh-Cyclopentylformiat kann ohne weitere Aufreinigung in der zweiten Reaktionsstufe eingesetzt werden.

Gemäß einer Ausführungsform der Erfindung wird das Cyclopentan aus dem so gewonnenen Roh-Cyclopentylformiat entfernt, beispielsweise mittels Destillation. Dabei müssen nur geringe Mengen an Ameisensäure, die im Cyclopentan gelöst sind, abdestilliert werden, so daß Korrosionsprobleme in der Destillationsapparatur vermieden werden. Die bei der Extraktion anfallende ameisensäurereiche Phase kann direkt in die Umsetzung zurückgeführt werden.

Ebenso kann das bei der Destillation entfernte Cyclopentan in die Reaktion oder die Extraktion zurückgeführt werden oder abgeführt werden. Neben dem erfindungsgemäß zur Extraktion bevorzugten Cyclopentan kann auch jedes andere geeignete Extraktionsmittel verwendet werden. Vorzugsweise werden dabei solche Extraktionsmittel verwendet, die eine geringe Löslichkeit für Ameisensäure aufweisen.

Das erhaltene Cyclopentylformiat kann für eine Vielzahl von chemischen Umsetzungen eingesetzt werden.

Gemäß einer Ausführungsform der Erfindung wird das gebildete Cyclopentylformiat in Cyclopentanol überführt. Das Cyclopentylformiat kann beispielsweise mit wäßriger Lauge, insbesonders wäßriger Natronlauge hydrolysiert werden. Bei diesem Verfahren werden stöchiometrische Mengen an Natronlauge verbraucht und die gebildete Ameisensäure fällt in Form von Natriumformiat an.

Gemäß einer Ausführungsform der Erfindung werden die Cyclopentylformiate umgesetzt mit einer Verbindung der allgemeine Formel R⁴ₙXH wobei X ein Stickstoffatom ist und n den Wert 2 hat oder X ein Sauerstoffatom ist und n den Wert 1 hat und R⁴, gegebenenfalls unabhängig voneinander ein Wasserstoffatom, ein linearer oder verzweigter C₁₋₁₂-Alkylrest oder ein C₇₋₁₂-Aralkylrest ist.

Vorzugsweise ist R⁴ ein Wasserstoffatom, oder ein Methylrest, insbesondere Wasserstoffatom, wenn X Stickstoff ist. R⁴ ist vorzugsweise ein C₁₋₄-Alkylrest, wie Methyl-, Ethyl-, n-Propyl-, i-Propyl-, n-Butyl-, i-Butyl-, tert.-Butylrest, vorzugsweise ein Methylrest, insbesondere wenn X Sauerstoff ist.

Bei der Umsetzung mit Ammoniak, der entweder gasförmig oder als wäßrige Lösung eingesetzt werden kann, oder Aminen werden Formamide gebildet.

Erfindungsgemäß bevorzugt ist die Umsetzung des Cyclopentylformiats mit Methanol, vorzugsweise in Gegenwart eines Umesterungskatalysators. Dabei wird das Cyclopentylformiat zu Cyclopentanol umgesetzt und gleichzeitig Methanol zum Methylformiat. Als Katalysatoren für diese Umsetzung kommen erfindungsgemäß Basen, Säuren oder Metallkomplexe, wie beispielsweise Titanate oder Dibutylzinndilaurat in Betracht. Vorzugsweise werden Basen, wie insbesondere Natriummethanolat als Katalysator eingesetzt.

Das bei der Umsetzung gebildete Methylformiat kann aus dem Reaktionsgemisch leicht destillativ abgetrennt werden. Das so erhaltene Methylformiat wird gemäß einer Ausführungsform der Erfindung, gegebenenfalls in Gegenwart eines basischen Katalysators, zu Ameisensäure und Methanol hydrolysiert, die wieder in die Synthese zurückgeführt werden. Die Ameisensäure wird dabei vorzugsweise in die erste Umsetzung von Cyclopentenen mit Ameisensäure zurückgeführt, das Methanol in die Umesterung des Cyclopentylformiats zurückgeführt.

Insgesamt ergibt sich bei dieser Ausführungsform der Erfindung für beide Reaktionsschritte als Nettoreaktion die Addition von Wasser an Cyclopenten. Es entsteht kein Koppelprodukt, das neben dem gewünschten Produkt anfällt.

Die Umesterung mit Methanol verläuft nahezu quantitativ, so daß aus der Umsetzung sehr reines Cyclopentanol erhalten wird. Das Cyclopentanol kann gemäß einer Ausführungsform der Erfindung direkt eingesetzt werden in weiteren chemischen Umsetzungen. Dabei ist Cyclopentanol eine Zwischenstufe für die Synthese verschiedener Cyclopentanderivate. Es können beispielsweise nach bekannten Verfahren Cyclopentanon, Cyclopentylamin, Cyclopentylchlorid und Cyclopentylbromid wie auch Cyclopentylchlorformiat hergestellt werden. Werden sehr hohe Reinheitsgrade des Cyclopentanols benötigt, so kann das erhaltene Cyclopentanol beispielsweise destillativ weiter aufgereinigt werden.

Insbesondere beim Einsatz von C₅-Strömen aus einem (Steam)cracker oder einer Raffinerie, gegebenenfalls nach destillativer Abtrennung leichter flüchtiger Komponenten, können Cyclopentanole sehr wirtschaftlich hergestellt werden, da gleichzeitig im C₅-Strom vorliegendes Cyclopentan als Extraktionsmittel verwendet wird und die verwendeten chemischen Verbindungen in die Umsetzung zurückgeführt werden können. Insbesondere das bei der Umesterung entstehende Methylformiat kann in die Ameisensäuresynthese zurückgeführt werden oder nach separater Esterspaltung in den ersten bzw. zweiten Umsetzungsschritt zurückgeführt werden.

Die Erfindung wird nachstehend anhand von Beispielen erläutert.

### BEISPIELE 1 bis 7

### Umsetzung von Cyclopenten mit Ameisensäure

Cyclopenten (96%ig, 35,4 g, 0,5 mol), Ameisensäure (99%ig, 92 g, 2 mol) und der in der nachstehenden Tabelle angegebene Katalysator (5 mmol, 1 mol%) wurden in einen Glasautoklaven eingefüllt und unter guter Rührung drei Stunden bei 80°C unter Eigendruck erhitzt. Nach dem Abkühlen wurden Selektivität und Umsatz durch quantitative Gaschromatographie bestimmt.
Die Ergebnisse sind in der nachstehenden Tabelle zusammengefaßt.

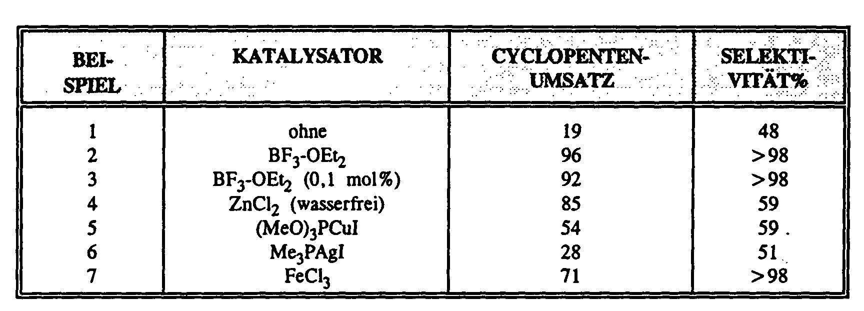

Aus den Ergebnissen der Tabelle geht hervor, daß Cyclopenten in hoher Ausbeute und mit hoher Selektivität, insbesondere bei Verwendung eines Katalysators, speziell BF₃·OEt₂, zu Cyclopentylformiat umgesetzt werden kann.

### Vergleichsbeispiel 1

Zu Vergleichszwecken wurden die von Wunderly und Sowa gemäß dem Stand der Technik eingesetzen Edukte Cyclohexen und Essigsäure gemäß Beispiel 2 umgesetzt. Dazu wurden Cyclohexen (41g, 0,05 mol), Essigsäure (120g, 2 mol) und BF₃-etherat (5mmol) analog Beispiel 2 in einen Glasautoklaven eingefüllt und unter guter Rührung drei Stunden bei 80° C unter Eigendruck erhitzt. Nach dem Abkühlen wurden Selektivität und Umsatz durch quantitative Gaschromatographie bestimmt. Es ergab sich ein Cyclohexenumsatz von 12% bei einer Selektivität von mehr als 98%.

### BEISPIEL 8

### Umsetzung eines cyclopentenhaltigen Gemisches mit Ameisensäure

Ein cyclopentenhaltiges Gemisch aus einem C₅-Schnitt eines Steamcrackers (50 g, 49 Gew.-% Cyclopenten, 0,36 mol, weitere Zusammensetzung siehe Tabelle in der Beschreibung) wurde mit Ameisensäure (99%ig, 76 g, 1,44 mol) und BF₃-Etherat (41 mg, 0,36 mol) in einem Glasautoklaven eingefüllt und unter guter Rührung 4 Stunden bei 80°C unter Eigendruck erhitzt. Der Cyclopentenumsatz betrug 92%, wie mittels quantitativer GC-Analyse bestimmt wurde. Die Selektivität zu Cyclopentylformiat betrug dabei mehr als 98%.

### Vergleichsbeispiel 2

### Umsetzung von Cyclohexen mit Essigsäure

Umsetzung eines Cyclohexen/Cyclohexan-Gemisches mit Essigsäure. Zu Vergleichszwecken wurden die von Wunderly und Sowa verwendeten Edukte Essigsäure und Cyclohexen in Form eines Cyclohexen/Cyclohexan-Gemisches analog Beispiel 8 umgesetzt. Cyclohexen (20,5g, 0,025 mol), Cyclohexan (21g, 0,025 mol), Essigsäure (60g, 1 mol) und BF₃-Etherat (2,5 mmol) wurden in einen Glasautoklaven eingefüllt und unter guter Rührung vier Stunden bei 80° C unter Eigendruck erhitzt. Nach dem Abkühlen wurden Selektivität und Umsatz durch quantitative Gaschromatographie bestimmt. Der Cyclohexenumsatz betrug 5% bei einer Selektivität von mehr als 98%.

### BEISPIEL 9

### Extraktion von Cyclopentylformiat

500 ml Reaktionsaustrag aus Beispiel 8 wurden mehrmals mit jeweils 500 ml Cyclopentan bzw. n-Pentan extrahiert. Die kumulierten Extraktionsausbeuten nach jeder Extraktionsstufe sind in der nachstehenden Tabelle zusammengefaßt.

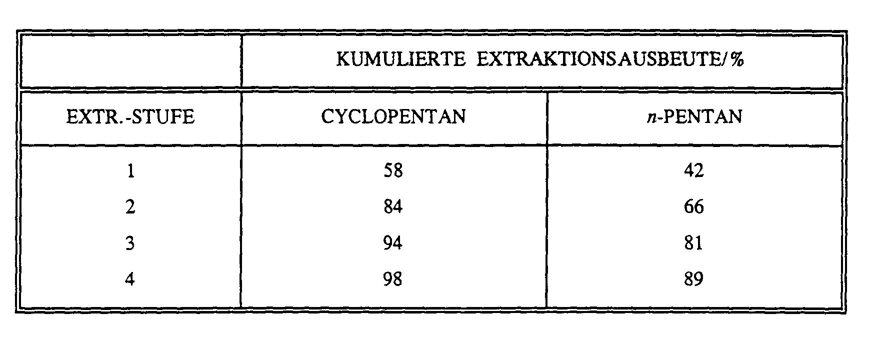

In beiden Fällen enthält das gewonnene Cyclopentylformiat noch etwa 5 Gew.-% Ameisensäure. Die Extraktion mit Cyclopentan ist deutlich besser.

### BEISPIEL 10

### Umesterung von Cyclopentylformiat mit Methanol zu Cyclopentanol und Methylformiat

Cyclopentylformiat (0,08 mol) wurde mit 100 ml Methanol und 0,8 mmol NaOCH₃ unter Rühren zum Sieden erhitzt. Das gebildete Methylformiat wurde über eine Destillationskolonne bis zum Erreichen einer Übergangstemperatur von 65°C (Siedepunkt von Methanol) abdestilliert. Die Ausbeute an Cyclopentanol wurde mittels quantitativer Gaschromatographie bestimmt und betrug 96%.

## Patentansprüche

1. Verfahren zur Herstellung von Cyclopentylformiaten der allgemeinen Formel (II) 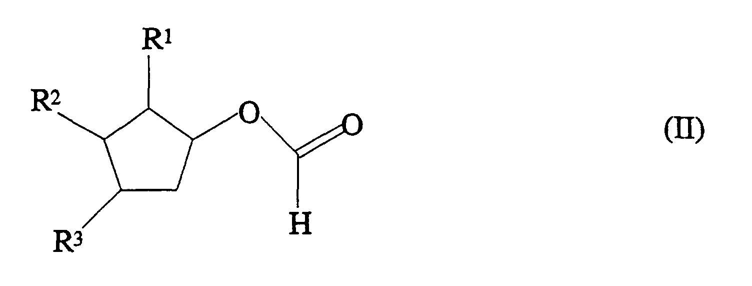 durch Umsetzung von Cyclopentenen der allgemeinen Formel (I) 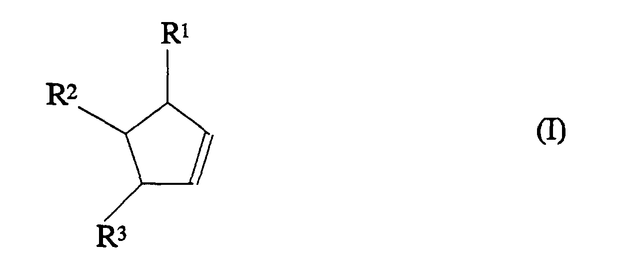 wobei R¹, R² und R³ unabhängig voneinander Wasserstoffatome oder C₁₋₈-Alkylreste sind, mit Ameisensäure in Gegenwart eines Säure-Katalysators, der ausgewählt ist aus Lewis-Säuren der allgemeinem Formeln BX₃, AlX₃, SnX₄, FeX₃, MgX₂, ZnX₂, wobei X ein Halogenid oder der Rest C₆F₅ ist, Metallkomplexen der allgemeinen Formel (R₃P) MY, wobei M Kupfer, Silber oder Gold bedeutet, Y ein Halogenid, Nitrat, Sulfat, Carboxylat oder Tosylat und R ein C₁₋₁₂-Alkylrest, C₆₋₁₂-Arylrest, C₁₋₁₂-O-Alkylrest oder C₆₋₁₂-O-Arylrest ist.

2. Verfahren nach Anspruch 1, **dadurch gekennzeichnet, daß** alle Reste Wasserstoffatome oder 1 bis 3 der Reste R¹, R² und R³ Methylreste sind und die übrigen Reste Wasserstoffatome sind.

3. Verfahren nach Anspruch 1 oder 2, **dadurch gekennzeichnet, daß** das Cyclopenten der allgemeinen Formel (I) in Form eines C₅-Stroms aus einem Cracker oder einer Raffinerie eingesetzt wird.

4. Verfahren nach einem der vorstehenden Ansprüche, **dadurch gekennzeichnet, daß** die Cyclopentylformiate durch Extraktion des Reaktionsgemisches mit Cyclopentan und anschließendes Entfernen des Cyclopentans erhalten werden.

5. Verfahren nach Anspruch 4, **dadurch gekennzeichnet, daß** zur Extraktion das im C₅-Strom eines Crackers oder einer Raffinerie vorliegende Cyclopentan verwendet wird.

6. Verfahren zur Herstellung von Cyclopentanolen der allgemeinen Formel (III) 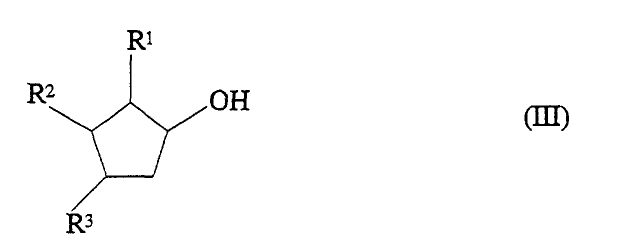 durch Umsetzung der nach einem Verfahren gemäß einem der Ansprüche 1 bis 5 erhaltenen Cyclopentylformiate mit einer Verbindung der allgemeinen Formel R⁴ₙXH,
wobei X ein Stickstoffatom ist und n den Wert 2 hat oder X ein Sauerstoffatom ist und n den Wert 1 hat
und R⁴, gegebenenfalls unabhängig voneinander ein Wasserstoffatom, ein linearer oder verzweigter C₁₋₁₂-Alkylrest oder ein C₇₋₁₂-Aralkylrest ist.

7. Verfahren nach Anspruch 6, **dadurch gekennzeichnet, daß** X ein Sauerstoffatom und R⁴ ein C₁₋₄-Alkylrest ist.

8. Verfahren nach Anspruch 7, **dadurch gekennzeichnet, daß** die Cyclopentylformiate mit Methanol in Gegenwart eines Umesterungskatalysators umgeestert werden und das bei der Umesterung erhaltene Methylformiat zu Ameisensäure und Methanol hydrolysiert wird, die wieder in die Synthese zurückgeführt werden.

## Claims

1. A process for preparing cyclopentyl formates of the formula (II) 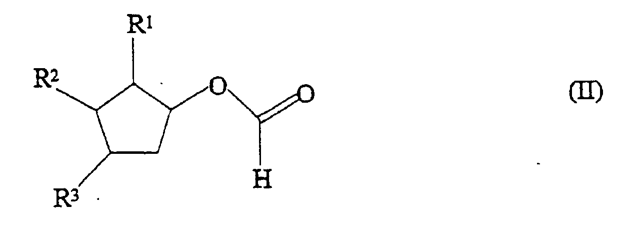 by reacting cyclopentenes of the formula (I) 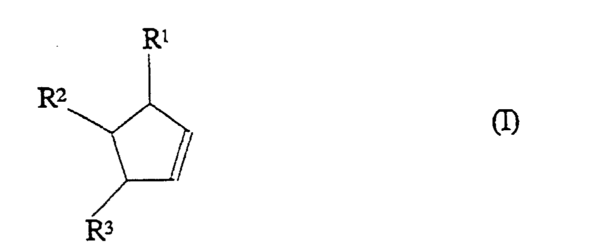 where R¹, R² and R³, independently of one another, are hydrogen atoms or C₁₋₈-alkyl,
with formic acid in the presence of an acid catalyst which is selected from Lewis acids of the formulae BX₃, AlX₃, SnX₄, FeX₃, MgX₂, ZnX₂, where X is a halide or the radical C₆F₅, metal complexes of the formula (R₃P) MY, where M is copper, silver or gold, Y is a halide, nitrate, sulfate, carboxylate or tosylate and R is a C₁₋₁₂-alkyl, C₆₋₁₂-aryl, C₁₋₁₂-O-alkyl or C₆₋₁₂-O-aryl.

2. A process as claimed in claim 1, wherein all the radicals are hydrogen or from 1 to 3 of the radicals R¹, R² and R³ are methyl and the other radicals are hydrogen.

3. A process as claimed in claim 1 or 2, wherein the cyclopentene of the formula (I) is used in the form of a C₅ stream from a cracker or a refinery.

4. A process as claimed in any one of the preceding claims, wherein the cyclopentyl formates are obtained by extraction of the reaction mixture with cyclopentane and subsequently removal of the cyclopentane.

5. A process as claimed in claim 4, wherein the extraction makes use of the cyclopentane-present in the C₅ stream of a cracker or a refinery.

6. A process for preparing cyclopentanols of the formula (III) 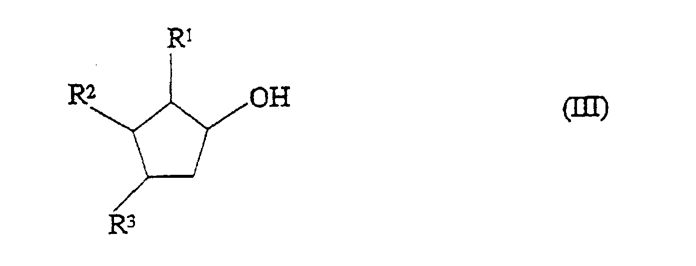 by reacting the cyclopentyl formates obtained in accordance with the process as claimed in any one of claims 1 to 5 with a compound of the formula R⁴ₙXH,
where X is nitrogen and n has the value 2 or X is oxygen and n has the value 1
and R⁴, possibly independently of each other, is hydrogen, a linear or branched C₁₋₁₂-alkyl or a C₇₋₁₂-aralkyl.

7. A process as claimed in claim 6, wherein X is oxygen and R⁴ is a C₁₋₄-alkyl.

8. A process as claimed in claim 7, wherein the cyclopentyl formates are transesterified with methanol in the presence of a transesterification catalyst and the methyl formate obtained in the transesterification is hydrolyzed to formic acid and methanol which are recycled into the synthesis.

## Revendications

1. Procédé de préparation de formiates de cyclopentyle de formule générale (II) 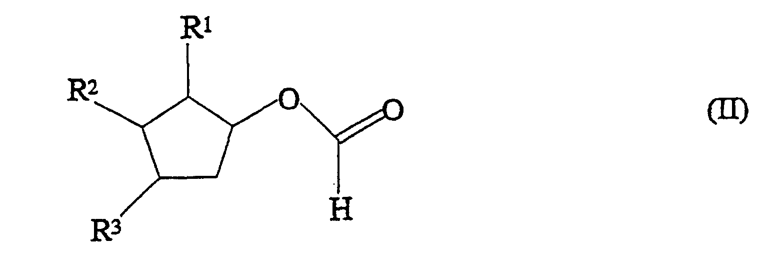 par réaction de cyclopentènes de formule générale (I) 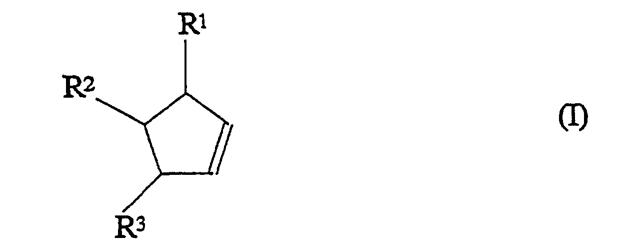 dans lesquelles R¹, R² et R³ représentent indépendamment les uns des autres, des atomes d'hydrogène ou des résidus alkyle en C₁ à C₈,
avec l'acide formique en présence d'un catalyseur acide, qui est choisi parmi les acides de Lewis de formules générales BX₃, AlX₃, SnX₄, FeX₃, MgX₂, ZnX₂, dans lesquelles X représente un halogénure ou le résidu C₆F₅, les complexes métalliques de formule générale (R₃P)MY, dans laquelle M représente un atome de cuivre, d'argent ou d'or, Y représente un halogénure, nitrate, sulfate, carboxylate ou tosylate et R représente un résidu alkyle en C₁ à C₁₂, aryle en C₆ à C₁₂, alkoxy en C₁ à C₁₂, ou aryloxy en C₆ à C₁₂.

2. Procédé selon la revendication 1, **caractérisé en ce que** tous les résidus représentent des atomes d'hydrogène ou bien 1 à 3 des résidus R¹, R² et R³ représentent des résidus méthyle et les résidus restants sont des atomes d'hydrogène.

3. Procédé selon la revendication 1 ou 2, **caractérisé en ce que** le cyclopentène de formule générale (I) est mis en oeuvre sous forme d'un courant de coupe en C₅ provenant d'un craqueur ou d'une raffinerie.

4. Procédé selon l'une quelconque des revendications précédentes, **caractérisé en ce que** les formiates de cyclopentyle sont obtenus en extrayant le mélange réactionnel avec le cyclopentane, puis en éliminant le cyclopentane.

5. Procédé selon la revendication 4, **caractérisé en ce que** le cyclopentane existant dans le courant de coupe en C₅ d'un craqueur ou d'une raffinerie est utilisé pour l'extraction.

6. Procédé pour préparer des cyclopentanols de formule générale III 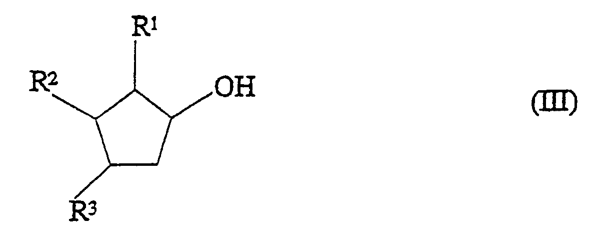 par réaction des formiates de cyclopentyle obtenus avec un procédé selon l'une quelconque des revendications 1 à 5, avec un composé de formule générale R⁴ₙXH,
dans laquelle X représente un atome d'azote, et n vaut 2, ou bien X représente un atome d'oxygène et n vaut 1,
et les R⁴, éventuellement, indépendamment les uns des autres, représentent chacun un atome d'hydrogène, un résidu alkyle linéaire ou ramifié en C₁ à C₁₂ ou un résidu aralkyle en C7 à C12

7. Procédé selon la revendication 6, **caractérisé en ce que** X représente un atome d'oxygène et R⁴ un résidu alkyle en C₁ à C₄.

8. Procédé selon la revendication 7, **caractérisé en ce que** les formiates de cyclopentyle sont transestérifiés avec le méthanol en présence d'un catalyseur de transestérification et que le formiate de méthyle obtenu dans la transestérification est hydrolysé en acide formique et en méthanol qui sont à nouveau recyclés dans la synthèse.
